# FASCICULE DE BREVET EUROPEEN

(11) **EP 3 060 552 B1**
(45) Date de publication et mention de la délivrance du brevet: **02.12.2020**
(21) Numéro de dépôt: 14790056.7
(22) Date de dépôt: 24.10.2014
(51) Int. Cl.: C07D 233/61, C08C 19/22, C08F 236/10, C08F 8/30

(54) **COMPOSÉ 1,3-DIPOLAIRE PORTANT UNE FONCTION IMIDAZOLE ET PROCÉDÉ POUR MODIFIER UN POLYMÈRE INSATURÉ PAR GREFFAGE**
1,3-DIPOLARE VERBINDUNG MIT EINER IMIDAZOLFUNKTION UND VERFAHREN ZUR MODIFIZIERUNG EINES UNGESÄTTIGTEN POLYMERS DURCH PFROPFUNG
1,3-DIPOLAR COMPOUND BEARING AN IMIDAZOLE FUNCTION AND PROCESS FOR MODIFYING AN UNSATURATED POLYMER BY GRAFTING

(30) Priorité: 25.10.2013 FR 1360413
(43) Date de publication de la demande: 31.08.2016
(73) Titulaire: Compagnie Générale des Etablissements Michelin, 63000 Clermont-Ferrand (FR)
(72) Inventeur: UGOLNIKOV, Oleg, F-63040 Clermont-Ferrand Cedex 9 (FR); IVANOV, Sergey, F-63040 Clermont-Ferrand Cedex 9 (FR); RANNOUX, Claire, F-63040 Clermont-Ferrand Cedex 9 (FR); SALIT, Anne-Frédérique, F-63040 Clermont-Ferrand Cedex 9 (FR); GANDER, Sophie, F-63040 Clermont-Ferrand Cedex 9 (FR); THUILLIEZ, Anne-Lise, F-63040 Clermont-Ferrand Cedex 9 (FR)
(74) Mandataire: Gandon-Pain, Sylvie
(86) Numéro de dépôt international: PCT/EP2014/072838
(87) Numéro de publication internationale: WO 2015/059269

(56) Documents cités:
- EP-A1- 0 310 061
- EP-A1- 0 945 426
- EP-A2- 0 257 289
- EP-A2- 0 257 391
- EP-A2- 0 373 549
- WO-A1-2012/007441
- WO-A2-2008/002614
- BRUNO CAVALLERI ET AL: "Synthesis and biological activity of some vinyl-substituted 2-nitroimidazoles", JOURNAL OF MEDICINAL CHEMISTRY, vol. 20, no. 5, 1 mai 1977 (1977-05-01), pages 656-660, XP055104751, ISSN: 0022-2623, DOI: 10.1021/jm00215a007

## Description

Le domaine de la présente invention est celui du greffage avec des agents de modification destinés à fonctionnaliser des polymères insaturés le long de la chaîne polymère.

La modification de la structure chimique d'un polymère impacte généralement les propriétés chimiques et physiques du polymère, ainsi que les propriétés des compositions le contenant. La modification de structure d'un polymère, telle que la fonctionnalisation d'un polymère, est particulièrement recherchée lorsque l'on souhaite mettre en présence un polymère et une charge dans une composition. La modification chimique d'un polymère peut améliorer la dispersion de la charge dans le polymère et permettre ainsi l'obtention d'un matériau davantage homogène. Dans le cas de certaines charges, comme le noir de carbone ou la silice, une meilleure dispersion de la charge va généralement se traduire par une baisse d'hystérèse de la composition. Une telle propriété est recherchée, notamment dans les compositions de caoutchouc destinées par exemple à des applications pour pneumatique. Cette baisse d'hystérèse s'accompagne souvent d'une baisse de la rigidité à cuit de la composition, ce qui peut rendre la composition inadaptée à l'usage que l'on veut en faire. Il existe donc un besoin de trouver des agents de modification qui permettent à la fois de fonctionnaliser un polymère et de modifier ce compromis hystérèse rigidité à cuit d'une composition comprenant un polymère et une charge.

Parmi les réactions chimiques pour modifier un polymère insaturé, on décompte les réactions de greffage d'un composé. Des composés connus pour se greffer sur un polymère insaturé sont par exemple des composés 1,3-dipolaires tels que décrits dans les demandes de brevet WO 2006/045088 et WO 2012/007441. La première demande décrit des composés qui permettent le greffage de fonction oxazoline, thiazoline, alcoxysilane ou allylétain. La seconde décrit des composés qui permettent le greffage de fonctions associatives azotées. Mais aucune de ces demandes ne décrit ni des composés 1,3-dipolaires portant un cycle imidazole, ni le greffage de cycle imidazole sur un polymère insaturé par réaction de ces composés 1,3-dipolaires dans le but de modifier le compromis hystérèse rigidité à cuit d'une composition comprenant le polymère en présence d'une charge.

L'invention concerne un procédé pour modifier un polymère insaturé, lequel procédé comprend la réaction d'un composé 1,3-dipolaire sur au moins une et de préférence plusieurs insaturations du polymère insaturé, le composé 1,3-dipolaire étant choisi dans le groupe constitué par les oxydes de nitrile, les imines de nitrile et les nitrone et répondant à la formule (I)

Q-A-B (I)

dans laquelle :
Q contient un motif -C≡N→O, -C≡N→N- ou -C=N(→O)-,
A est un atome ou un groupe d'atomes reliant Q à B,
B comprend un cycle répondant à la formule (II) 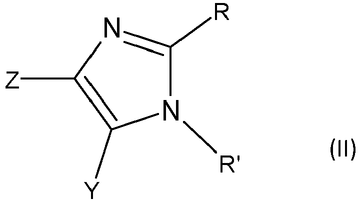
dans laquelle :
3 des 4 symboles Z, Y, R et R' identiques ou différents représentent chacun un atome ou un groupe d'atomes,
et le quatrième symbole Z, Y, R ou R' désigne un rattachement direct à A,
dans lequel :
A contenant jusqu'à 20 atomes de carbone est un groupe aliphatique ou un groupe aromatique,
R' désigne le rattachement direct à A,
R représente un atome d'hydrogène ou un groupe alkyle,
Z et Y sont chacun un atome d'hydrogène.

L'invention concerne également un polymère qui est susceptible d'être obtenu par le procédé conforme à l'invention.

L'invention concerne aussi un composé 1,3-dipolaire défini dans l'une quelconque des revendications 2 à 6 et un composé 1,3-dipolaire défini dans l'une quelconque des revendications 8 à 9, lesquels composés sont utiles au procédé de greffage conforme à l'invention.

### I. DESCRIPTION DETAILLEE DE L'INVENTION

Dans la présente description, sauf indication expresse différente, tous les pourcentages (%) indiqués sont des % en masse. L'abréviation "pce" signifie parties en poids pour cent parties d'élastomère (du total des élastomères si plusieurs élastomères sont présents).

D'autre part, tout intervalle de valeurs désigné par l'expression "entre a et b" représente le domaine de valeurs supérieur à "a" et inférieur à "b" (c'est-à-dire bornes a et b exclues) tandis que tout intervalle de valeurs désigné par l'expression "de a à b" signifie le domaine de valeurs allant de "a" jusqu'à "b" (c'est-à-dire incluant les bornes strictes a et b).

Le terme composé 1,3-dipolaire est compris selon la définition donnée par IUPAC.

Le composé 1,3-dipolaire utile au procédé conforme à l'invention répond à la formule (I) :

Q-A-B (I)

dans laquelle :
Q contient un motif -C≡N→O, -C≡N→N- ou -C=N(→O)-,
A est un atome ou un groupe d'atomes reliant Q à B,
B comprend un cycle imidazole répondant à la formule (II) : 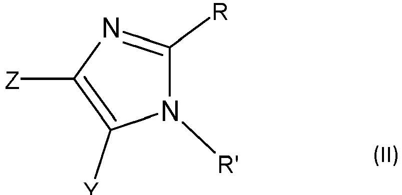
dans laquelle :
3 des 4 symboles Z, Y, R et R' identiques ou différents représentent chacun un atome ou un groupe d'atomes,
et le seul quatrième symbole désigne un rattachement direct à A.

Selon l'invention, R' désigne un rattachement direct à A, auquel cas R' est le 4^{ème} symbole.

Selon l'invention, Z et Y sont chacun un atome d'hydrogène.

Selon un mode de réalisation particulier de l'invention, R représente un atome d'hydrogène.

Selon un mode de réalisation particulier de l'invention, R est un groupe alkyle contenant de préférence de 1 à 12 atomes de carbone, de manière encore plus préférentielle un méthyle.

A est un groupe contenant jusqu'à 20 atomes de carbone, lequel groupe peut contenir au moins un hétéroatome. A est un groupe aliphatique ou aromatique.

Lorsque A est un groupe aliphatique, A contient préférentiellement de 1 à 20 atomes de carbone, plus préférentiellement de 1 à 12 atomes de carbone, encore plus préférentiellement de 1 à 6 atomes de carbone, tout particulièrement de 1 à 3 atomes de carbone. Lorsque A est un groupe aromatique, A contient préférentiellement de 6 à 20 atomes de carbone, plus préférentiellement de 6 à 12 atomes de carbone.

Comme groupe divalent A convient particulièrement un groupe alkylène contenant de 1 à 20 atomes de carbone, préférentiellement de 1 à 12 atomes de carbone, plus préférentiellement de 1 à 6 atomes de carbone, encore plus préférentiellement de 1 à 3 atomes de carbone. On peut citer comme groupe A divalent contenant de 1 à 3 atomes de carbone qui convient le groupe méthylène.

Comme groupe divalent A peut aussi convenir un groupe arylène contenant de préférence de 6 à 20 atomes de carbone, plus préférentiellement de 6 à 12 atomes de carbone.

Conviennent tout particulièrement comme composés 1,3-dipolaires les composés choisis dans le groupe constitué par les oxydes de nitrile, les imines de nitrile et les nitrones, auquel cas Q contient un motif -C≡N→O, -C≡N→N- ou -C=N(→O)-.

Selon le mode de réalisation particulier de l'invention où Q comprend un motif -C≡N→O, Q de préférence comporte, de manière plus préférentielle représente le motif répondant à la formule (III) dans laquelle quatre des cinq symboles R1 à R5 identiques ou différents, sont chacun un atome ou un groupe d'atomes et le cinquième symbole désigne un rattachement direct à A, sachant que R1 et R5 sont tous les deux différents de H. Les quatre des cinq symboles R1 à R5 peuvent être des groupes aliphatiques ou aromatiques. Les groupes aliphatiques peuvent contenir de 1 à 20 atomes de carbone, préférentiellement de 1 à 12 atomes de carbone, plus préférentiellement de 1 à 6 atomes de carbone, encore plus préférentiellement de 1 à 3 atomes de carbone. Les groupes aromatiques peuvent contenir de 6 à 20 atomes de carbone, préférentiellement de 6 à 12 atomes de carbone.

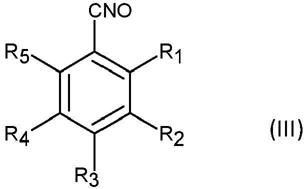

R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

Selon une variante de ce mode de réalisation particulier de l'invention, R1, R3 et R5 sont identiques. Selon cette variante où ils sont identiques, R1, R3 et R5 sont préférentiellement chacun un groupe alkyle de 1 à 6 atomes de carbone, plus préférentiellement de 1 à 3 atomes de carbone, encore plus préférentiellement un groupe méthyle ou éthyle.

De manière davantage préférentielle, le composé 1,3-dipolaire est le composé 2,4,6-triméthyl-3-((2-méthyl-1*H*-imidazol-1-yl)méthyl)benzonitrile oxyde répondant à la formule (IIIa) ou le composé 2,4,6-triéthyl-3-((2-méthyl-1*H*-imidazol-1-yl)méthyl)benzonitrile oxyde répondant à la formule (IIIb) :

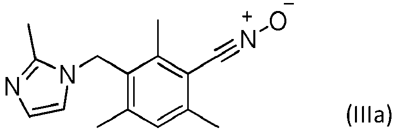

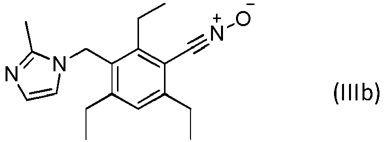

Selon le mode de réalisation particulier de l'invention où Q comprend un motif -C=N(→O)-, Q de préférence comporte, de manière plus préférentielle représente le motif répondant à la formule (IV) ou (V) 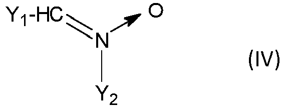 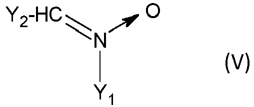 dans laquelle :
Y₁ est un groupe aliphatique, préférentiellement un groupe alkyle contenant de préférence 1 à 12 atomes de carbone, ou un groupe aromatique contenant de 6 à 20 atomes de carbone, préférentiellement un groupe alkylaryle, plus préférentiellement un groupe phényle ou tolyle,
et Y₂, comportant un rattachement direct à A, est un groupe aliphatique, préférentiellement un groupe alkylène contenant de préférence 1 à 12 atomes de carbone, ou un groupe aromatique contenant préférentiellement de 6 à 20 atomes de carbone et comportant sur son noyau benzénique le rattachement direct à A.

Le rattachement direct du noyau benzénique de Y₂ à A revient à dire que A est un substituant du noyau benzénique de Y₂.

Selon ce mode de réalisation particulier de l'invention, le composé 1,3-dipolaire est le composé de formule (IVa), (IVb), (Va) ou (Vb)

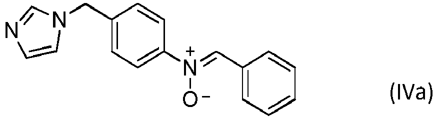

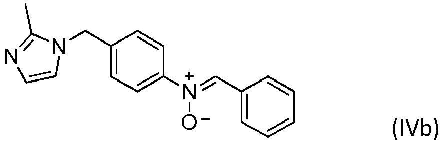

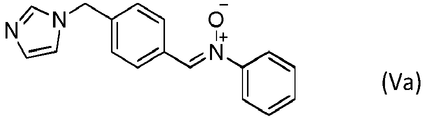

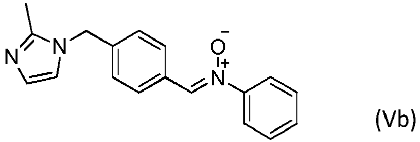

Le procédé, objet de l'invention, comprend la réaction du composé 1,3-dipolaire décrit précédemment sur au moins une et de préférence plusieurs insaturations d'un polymère insaturé. On entend par plusieurs insaturations au moins 2 insaturations.

Le composé 1,3-dipolaire peut être utilisé pour modifier un polymère insaturé par une réaction de greffage du composé 1,3-dipolaire sur au moins une et de préférence plusieurs insaturations du polymère insaturé.

Selon un mode de réalisation préférentiel de l'invention, les insaturations du polymère sont des liaisons carbone-carbone, de préférence des doubles liaisons carbone-carbone.

Le greffage du composé 1,3-dipolaire est effectué par cycloaddition [3+2] du ou des groupes réactifs du composé 1,3-dipolaire sur une ou plusieurs doubles liaisons d'une chaîne élastomère diénique. Le mécanisme de la cycloaddition d'un oxyde de nitrile, d'une nitrone et d'une imine de nitrile peut être illustré par les équations suivantes, dans lesquelles le symbole ¤ représente un quelconque substituant :
- Cycloaddition d'un oxyde de nitrile sur une insaturation ou double liaison d'un élastomère diénique (ici un polyisoprène) 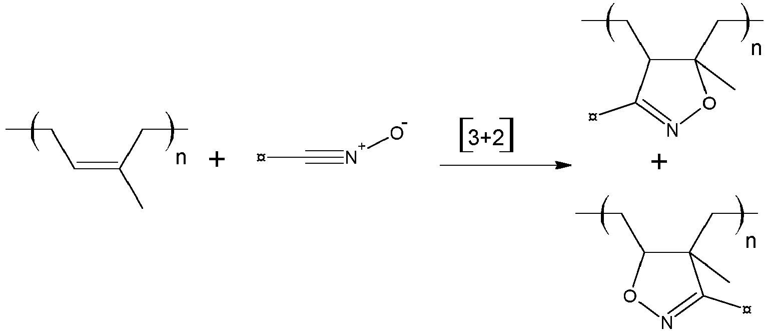
- Cycloaddition d'une nitrone sur une insaturation ou double liaison d'un élastomère diénique (ici un polyisoprène) 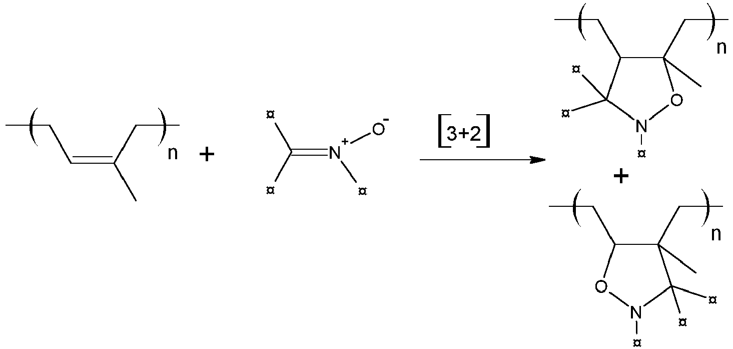
- Cycloaddition d'une imine de nitrile sur une insaturation ou double liaison d'un élastomère diénique (ici un polyisoprène)

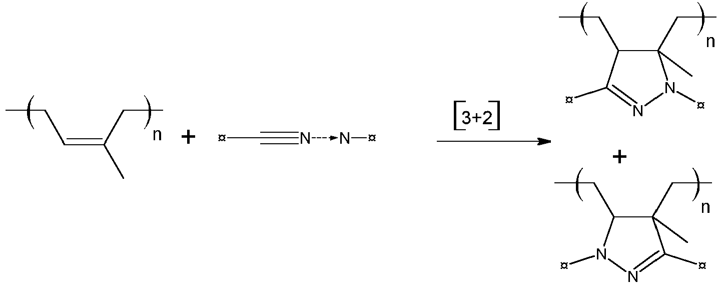

Le greffage du composé 1,3-dipolaire peut être réalisé en masse, par exemple dans un mélangeur interne ou un mélangeur externe tel qu'un mélangeur à cylindres. Le greffage est alors mis en œuvre soit à une température du mélangeur externe ou du mélangeur interne inférieure à 60°C, suivi d'une étape de réaction de greffage sous presse ou en étuve à des températures allant de 80°C à 200°C, soit à une température du mélangeur externe ou du mélangeur interne supérieure à 60°C sans traitement thermique postérieur.

Le procédé de greffage peut également être effectué en solution. La température à laquelle est mené le greffage est facilement ajustée par l'homme du métier à partir de ses connaissances générales en tenant compte de la concentration du milieu réactionnel, de la température de reflux du solvant, de la stabilité thermique du polymère et du composé 1,3-dipolaire. Par exemple peut convenir une température aux alentours de 60°C. Le polymère ainsi modifié peut être séparé de sa solution par tout type de moyen connu par l'homme de l'art et en particulier par une opération d'évaporation du solvant sous pression réduite ou par une opération de stripping à la vapeur d'eau.

Dans la réaction de greffage pour modifier le polymère insaturé, on fait réagir le composé 1,3-dipolaire selon une stœchiométrie préférentielle comprise entre 0 et 3 équivalents molaires, plus préférentiellement entre 0 et 2 équivalents molaires, encore plus préférentiellement entre 0 et 1 équivalent molaire, voire encore plus préférentiellement entre 0 et 0.7 équivalent molaire de cycle imidazole pour 100 moles d'unités monomères constituant le polymère. Pour chacune de ces plages préférentielles, la borne inférieure est avantageusement d'au moins 0.1 équivalent molaire de composé 1,3-dipolaire. La quantité de composé 1,3-dipolaire utilisée pour greffer le polymère est exprimée en équivalent molaire de cycle imidazole. Par exemple, si le composé 1,3-dipolaire contient un seul cycle imidazole de formule (II) telle que définie précédemment, à une mole de composé 1,3-dipolaire correspond une mole de cycle imidazole. Si le composé 1,3-dipolaire contient deux cycles imidazole de formule (II) telle que définie précédemment, à une mole de composé 1,3-dipolaire correspond deux moles de cycle imidazole. Dans ce dernier cas l'utilisation du composé 1,3-dipolaire selon un équivalent molaire de cycle imidazole correspond à une demi-mole de composé 1,3-dipolaire.

De manière préférentielle, que le greffage soit réalisé en solution ou en masse, le polymère est au préalable antioxydé pour prévenir une éventuelle dégradation de la macrostructure du polymère au cours de la réaction de greffage.

Le polymère insaturé à modifier présente au moins une et de préférence plusieurs insaturations qui sont susceptibles de réagir avec le composé 1,3 dipolaire conforme à l'invention.

Le polymère insaturé est de préférence un polymère diénique, plus préférentiellement un élastomère diénique.

Par polymère diénique, doit être compris un polymère comprenant des unités monomères diéniques, en particulier des unités de monomères 1,3-diéniques.

Par élastomère (ou indistinctement caoutchouc) "diénique", doit être compris de manière connue un élastomère constitué au moins en partie (i.e., un homopolymère ou un copolymère) d'unités monomères diènes (monomères porteurs de deux doubles liaisons carbone-carbone, conjuguées ou non).

Ces élastomères diéniques peuvent être classés dans deux catégories : "essentiellement insaturés" ou "essentiellement saturés". On entend en général par "essentiellement insaturé", un élastomère diénique issu au moins en partie de monomères diènes conjugués, ayant un taux de motifs ou unités d'origine diénique (diènes conjugués) qui est supérieur à 15% (% en moles) ; c'est ainsi que des élastomères diéniques tels que les caoutchoucs butyle ou les copolymères de diènes et d'alpha-oléfines type EPDM n'entrent pas dans la définition précédente et peuvent être notamment qualifiés d'élastomères diéniques "essentiellement saturés" (taux de motifs d'origine diénique faible ou très faible, toujours inférieur à 15%). Dans la catégorie des élastomères diéniques "essentiellement insaturés", on entend en particulier par élastomère diénique "fortement insaturé" un élastomère diénique ayant un taux de motifs d'origine diénique (diènes conjugués) qui est supérieur à 50%.

Ces définitions étant données, on entend plus particulièrement par élastomère diénique susceptible d'être utilisé dans les compositions conformes à l'invention:
(a) - tout homopolymère d'un monomère diène conjugué, notamment tout homopolymère obtenu par polymérisation d'un monomère diène conjugué ayant de 4 à 12 atomes de carbone;
(b) - tout copolymère obtenu par copolymérisation d'un ou plusieurs diènes conjugués entre eux ou avec un ou plusieurs composés vinyle aromatique ayant de 8 à 20 atomes de carbone;
(c) - un copolymère ternaire obtenu par copolymérisation d'éthylène, d'une α-oléfine ayant 3 à 6 atomes de carbone avec un monomère diène non conjugué ayant de 6 à 12 atomes de carbone, comme par exemple les élastomères obtenus à partir d'éthylène, de propylène avec un monomère diène non conjugué du type précité tel que notamment l'hexadiène-1,4, l'éthylidène norbornène, le dicyclopentadiène;
(d) - un copolymère d'isobutène et d'isoprène (caoutchouc butyle), ainsi que les versions halogénées, en particulier chlorées ou bromées, de ce type de copolymère.

Bien qu'elle s'applique à tout type d'élastomère diénique, l'homme du métier du pneumatique comprendra que la présente invention est de préférence mise en œuvre avec des élastomères diéniques essentiellement insaturés, en particulier du type (a) ou (b) ci-dessus.

Dans le cas de copolymères du type (b), ceux-ci contiennent de 20 à 99% en poids d'unités diéniques et de 1 à 80% en poids d'unités vinylaromatique.

A titre de diènes conjugués conviennent notamment le butadiène-1,3, le 2-méthyl-1,3-butadiène, les 2,3-di(alkyle en C₁-C₅)-1,3-butadiènes tels que par exemple le 2,3-diméthyl-1,3-butadiène, le 2,3-diéthyl-1,3-butadiène, le 2-méthyl-3-éthyl-1,3-butadiène, le 2-méthyl-3-isopropyl-1,3-butadiène, un aryl-1,3-butadiène, le 1,3-pentadiène, le 2,4-hexadiène.

A titre de composés vinylaromatiques conviennent par exemple le styrène, l'ortho-, méta-, para-méthylstyrène, le mélange commercial "vinyle-toluène", le para-tertiobutylstyrène, les méthoxystyrènes, les chlorostyrènes, le vinylmésitylène, le divinylbenzène, le vinylnaphtalène.

Préférentiellement, l'élastomère diénique est un élastomère essentiellement insaturé choisi dans le groupe constitué par les polybutadiènes (BR), les polyisoprènes, les copolymères de butadiène, les copolymères d'isoprène, et les mélanges de ces élastomères. A titre d'élastomère diénique convient tout particulièrement un polybutadiène (BR), un copolymère de butadiène et de styrène (SBR), un caoutchouc naturel (NR) ou un polyisoprène de synthèse (IR) présentant préférentiellement un taux molaire de liaison cis-1,4 supérieur à 90%.

L'invention a également pour objet le polymère qui peut être obtenu par le procédé décrit selon l'un quelconque de ses modes de réalisation.

Le polymère, objet de l'invention, est de préférence un polymère diénique, préférentiellement un élastomère diénique, plus préférentiellement un élastomère diénique essentiellement insaturé, encore plus préférentiellement un élastomère essentiellement insaturé choisi dans le groupe constitué par les polybutadiènes, les polyisoprènes, les copolymères de butadiène, les copolymères d'isoprène et les mélanges de ces élastomères.

Un autre objet de l'invention est aussi un composé 1,3-dipolaire défini dans l'une quelconque des revendications 2 à 6.

Un autre objet de l'invention est un composé 1,3-dipolaire défini dans l'une quelconque des revendications 8 à 9.

Les caractéristiques précitées de la présente invention, ainsi que d'autres, seront mieux comprises à la lecture de la description suivante de plusieurs exemples de réalisation de l'invention, donnés à titre illustratif et non limitatif.

### II. EXEMPLES DE REALISATION DE L'INVENTION

### II.1-Mesures et tests utilisés :

### Analyse RMN :

L'analyse structurale ainsi que la détermination des puretés molaires des molécules de synthèses sont réalisées par une analyse RMN. Les spectres sont acquis sur un spectromètre Avance 3 400 MHz BRUKER équipé d'une sonde " large bande " BBFO-zgrad 5 mm. L'expérience RMN ¹H quantitative, utilise une séquence simple impulsion 30° et un délai de répétition de 3 secondes entre chacune des 64 acquisitions. Les échantillons sont solubilisés dans un solvant deutéré, le diméthylsulfoxide deutéré (DMSO) sauf indication contraire. Le solvant deutéré est également utilisé pour le signal de lock. Par exemple, la calibration est réalisée sur le signal des protons du DMSO deutéré à 2.44 ppm par rapport à une référence TMS à 0ppm. Le spectre RMN ¹H couplé aux expériences 2D HSQC ¹H/13C et HMBC ¹H/¹³C permettent la détermination structurale des molécules (cf tableaux d'attributions). Les quantifications molaires sont réalisées à partir du spectre RMN 1D ¹H quantitatif.

La détermination du taux molaire de composé oxyde de nitrile greffé est réalisée par une analyse RMN. Les spectres sont acquis sur un spectromètre 500 MHz BRUKER équipé d'une « CryoSonde BBFO-zgrad-5 mm ». L'expérience RMN ¹H quantitative, utilise une séquence simple impulsion 30° et un délai de répétition de 5 secondes entre chaque acquisition. Les échantillons sont solubilisés dans le chloroforme deutéré (CDCl3) dans le but d'obtenir un signal de « lock ».

Des expériences RMN 2D ont permis de vérifier la nature du motif greffé grâce aux déplacements chimiques des atomes de carbone et de proton.

### II.2-Synthèse du composé 1,3-dipolaire 2,4,6-triméthyl-3-((2-méthyl-1H-imidazol-1-yl)méthyl)benzonitrile oxyde :

Ce composé peut être préparé selon le schéma réactionnel suivant :

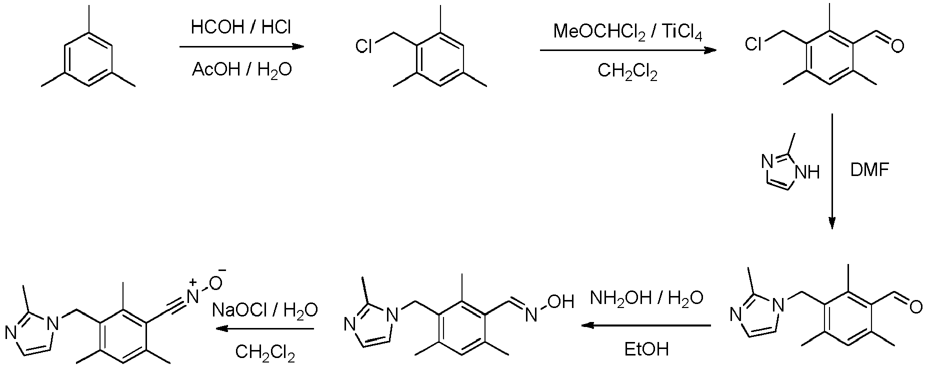

### 11.2-1-Synthèse du 2-(chlorométhyl)-1,3,5-triméthylbenzène :

Ce composé peut être obtenu selon une procédure décrite dans l'article suivant : Zenkevich, I. G.; Makarov, A. A.; Russian Journal of General Chemistry; vol. 77; nb. 4; (2007); p. 611 -619 (Zhurnal Obshchei Khimii; vol. 77; nb. 4; (2007); p. 653 - 662)

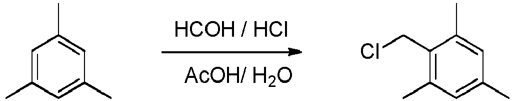

Un mélange de mésitylène (100,0 g, 0,832 mol), de para-formaldéhyde (26,2 g, 0,874 mol) et d'acide chlorhydrique (240 ml, 37 %, 2,906 mol) dans l'acide acétique (240 ml) est agité et chauffé très lentement (1,5 heures) jusqu'à 37°C. Après retour à température ambiante, le mélange est dilué par de l'eau (1,0 I) avec CH₂Cl₂ (200 ml), le produit est extrait par CH₂Cl₂ (4 fois par 50 mL). Les phases organiques sont rassemblées, puis lavées par l'eau (5 fois par 100 ml) et évaporées jusqu'à 11-12 mbar (Température du bain = 42°C). Une huile incolore (133,52 g, rendement 95 %) est obtenue. Après 15-18 heures à +4°C, l'huile a cristallisé. Les cristaux sont filtrés, lavés par de l'éther de pétrole refroidi à -18°C (40 ml), puis séchés pendant 3 à 5 heures sous pression atmosphérique à température ambiante. Un solide blanc (95,9 g, rendement 68 %) de point de fusion 39 °C est obtenu. La pureté molaire est supérieure à 96 % (RMN 1H).

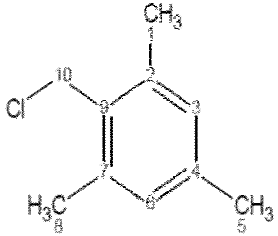

| **N°** | **δ¹H(ppm)** | **δ¹³C(ppm)** |
|---|---|---|
| 1-8 | 2,27 | 18,4 |
| 2-7 | - | 136,9 |
| 3-6 | 6,81 | 128,5 |
| 4 | - | 137,4 |
| 5 | 2,15 | 20,3 |
| 9 | - | 130,5 |
| 10 | 4,69 | 41,3 |

### II.2-2-Synthèse du 3-(chlorométhyl)-2,4,6-triméthylbenzaldéhyde:

Ce composé peut être obtenu selon une procédure décrite dans l'article suivant : Yakubov, A. P.; Tsyganov, D. V.; Belen'kii, L. I.; Krayushkin, M. M.; Bulletin of the Academy of Sciences of the USSR, Division of Chemical Science (English Translation); vol. 40; nb. 7.2; (1991); p. 1427 - 1432 (Izvestiya Akademii Nauk SSSR, Seriya Khimicheskaya; nb. 7; (1991); p. 1609 - 1615)

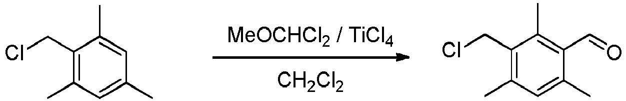

A une solution de TiCl₄ (90,0 g, 0,474 mol) dans le dichlorométhane (200 ml) à 17°C est ajoutée sous argon pendant 10-12 minutes une solution du 2-(chlorométhyl)-1,3,5-triméthylbenzène (20,0 g, 0,118 mol) et de dichlorométhylméthyl éther (27,26 g, 0,237 mol) dans le dichlorométhane (200 ml). Après agitation pendant 15-20 minutes à 17-20 °C, de l'eau (1000 ml) et de la glace (500 g) sont ajoutées au milieu réactionnel. Après 10-15 minutes d'agitation, la phase organique est séparée. La phase aqueuse est extraite par CH₂Cl₂ (3 fois par 75 ml). Les phases organiques rassemblées sont lavées par l'eau (4 fois par 100 ml) et évaporées sous pression réduite pour conduire à un solide (Température du bain = 28°C). Le produit cible (22,74 g) est obtenu avec un rendement de 97 % de point de fusion 58 °C. La pureté molaire estimée par RMN ¹H est de 95%mol.

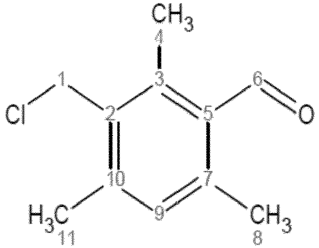

| **N°** | **δ¹H(ppm)** | **δ¹³C(ppm)** |
|---|---|---|
| 1 | 4,77 | 40,6 |
| 2 | - | 132,9 |
| 3 | - | 139,5 |
| 4 | 2,51 | 14,4 |
| 5 | - | 131,4 |
| 6 | 10,43 | 194,2 |
| 7 | - | 140,1 |
| 8 | 2,41 | 19,3 |
| 9 | 6,99 | 131,2 |
| 10 | - | 142,4 |
| 11 | 2,34 | 19,8 |

### II.2-3-Synthèse du 2,4,6-triméthyl-3-((2-méthyl-1H-imidazol-1-yl)méthyl) benzaldéhyde :

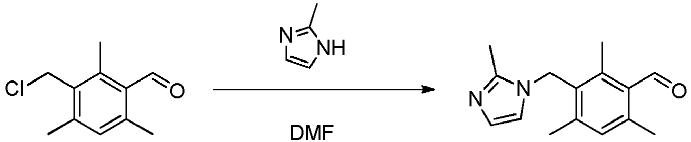

Un mélange de 3-(chlorométhyl)-2,4,6-triméthylbenzaldéhyde (10,0 g, 0,051 mol) et d'imidazole (10,44 g, 0,127 mol) dans le DMF (10 ml) est agité à 80°C pendant une heure. Après retour à 40-50°C, le mélange est dilué par l'eau (200ml), et agité pendant 10 minutes. Le précipité obtenu est filtré et lavé sur le filtre par l'eau (4 fois par 25 ml) puis séché à température ambiante. Un solide blanc (7,92 g, rendement 64 %) de point de fusion 161 °C est obtenu. La pureté molaire est 91 % (RMN ¹H).

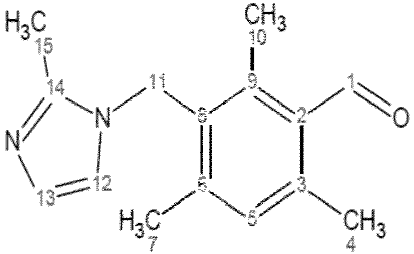

| **N°** | **δ¹H(ppm)** | **δ ¹³C (ppm)** |
|---|---|---|
| 1 | 10,45 | 194,2 |
| 2 | - | 131,5 |
| 3 | - | 139,5 |
| 4 | 2,44 | 19,6 |
| 5 | 7,04 | 131,2 |
| 6 | - | 142,5 |
| 7 | 2,19 | 19,5 |
| 8 | - | 131 |
| 9 | - | 139,5 |
| 10 | 2,34 | 14,6 |
| 11 | 5,02 | 42,5 |
| 12 | 6,24 | 116,9 |
| 13 | 6,59 | 125,9 |
| 14 | - | 143,5 |
| 15 | 2,32 | 12,7 |

### II.2-4-Synthèse du 2,4,6-triméthyl-3-((2-méthyl-1H-imidazol-1-yl)méthyl)benzaldéhyde oxime :

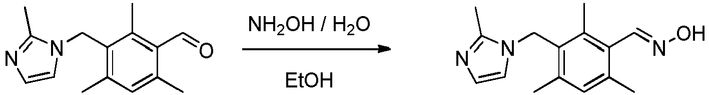

A une solution de 2,4,6-triméthyl-3-((2-méthyl-1H-imidazol-1-yl)méthyl) benzaldéhyde (20.3 g, 0,084mol) dans EtOH (110 ml) à 40°C, est ajoutée une solution aqueuse d'hydroxylamine (809 g, 0,134 mol, 50 % dans l'eau, Aldrich) dans EtOH (10 ml). Le milieu réactionnel est agité pendant 2,5 heures à une température de 50 à 55°C. Après retour à 23°C, le précipité obtenu est filtré et lavé deux fois sur le filtre par un mélange EtOH/H₂O (10 ml/15 ml) et séché pendant 15 à 20 heures sous pression atmosphérique à température ambiante. Un solide blanc (19.57 g, rendement 91 %) de point de fusion 247°C est obtenu. La pureté molaire est supérieure à 87 % (RMN ¹H).

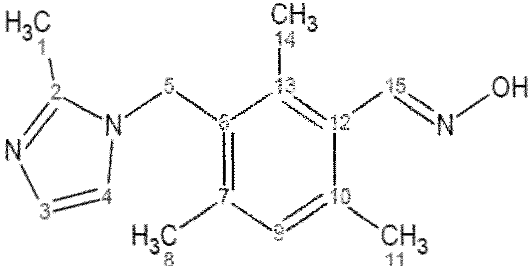

| **N°** | **δ¹H(ppm)** | **δ ¹³C (ppm)** |
|---|---|---|
| 1 | 2,31 | 12,7 |
| 2 | - | 143,4 |
| 3 | 6,58 | 125,8 |
| 4 | 6,22 | 116,9 |
| 5 | 4,97 | 43,2 |
| 6 | - | 129,3 |
| 7 | - | 136,2 |
| 8 | 2,23 | 20,2 |
| 9 | 6,97 | 130 |
| 10 | - | 137,3 |
| 11 | 2,15 | 19,1 |
| 12 | - | 129,1 |
| 13 | - | 136,1 |
| 14 | 2,11 | 15,9 |
| 15 | 8,25 | 147,4 |
| OH | 11,11 | - |

### II.2-5-Synthèse du 2,4,6-triméthyl-3-((2-méthyl-1H-imidazol-1-yl)méthyl)benzonitrile oxyde :

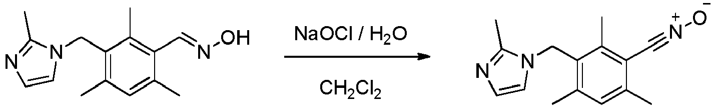

A un mélange de 2,4,6-triméthyl-3-((2-méthyl-1H-imidazol-1-yl)méthyl)benzaldéhyde oxime (8,80 g, 0,034 mol) dans CH₂Cl₂ (280 ml) à 6°C est ajoutée goutte à goutte une solution aqueuse de NaOCl (4 % de chlore actif, Aldrich, 49 ml) pendant 5 minutes. La température du milieu réactionnel est maintenue entre 6 et 8°C. Le milieu réactionnel est ensuite agité pendant 2 heures de 8°C à 21°C. La phase organique est séparée. La phase organique est lavée par l'eau (3 fois par 50 ml). Après concentration sous pression réduite (température du bain = 22-23°C, 220 mbar), de l'éther de pétrole (10 ml) est ajouté, le solvant est évaporé jusqu'à 8-10 ml, et la solution est maintenue à -18°C pendant 10-15 heures de façon à obtenir un précipité. Le précipité est filtré et lavé sur le filtre par le mélange de CH₂Cl₂ / éther de pétrole (2 ml / 6 ml) puis par l'éther de pétrole (2 fois 10 ml) et enfin séché pendant 10-15 heures sous pression atmosphérique à température ambiante. Un solide blanc (5,31 g, rendement 61 %) de point de fusion 139 °C est obtenu.

La pureté molaire est supérieure à 95 % mol (RMN ¹H).

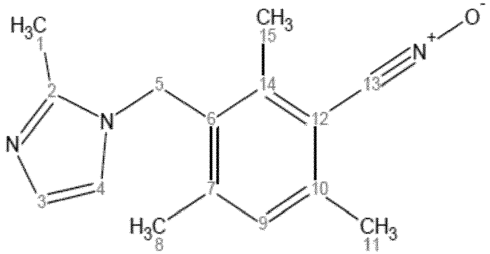

| **N°** | **δ¹H(ppm)** | **δ ¹³C(ppm)** |
|---|---|---|
| 1 | 2,3 | 12,6 |
| 2 | - | 143,6 |
| 3 | 6,59 | 126,1 |
| 4 | 6,27 | 117,1 |
| 5 | 4,99 | 43 |
| 6 | - | 130,6 |
| 7 | - | 140,7 |
| 8 | 2,16 | 19,2 |
| 9 | 7,12 | 129,9 |
| 10 | - | 141 |
| 11 | 2,34 | 20 |
| 12 | - | 112,1 |
| 13 | - | NI |
| 14 | - | 140,8 |
| 15 | 2,28 | 17,7 |

### II.3-Greffage du composé 1,3-dipolaire 2,4,6-triméthyl-3-((2-méthyl-1H-imidazol-1-yl)méthyl)benzonitrile oxyde :

On utilise l'agent de modification obtenu selon le mode opératoire décrit ci-dessus avec une pureté molaire de 93% molaire.

Le SBR avant modification contient 25% de motif styrène et 58% de motif 1,2 de la partie butadiénique.
On incorpore le 2,4,6-triméthyl-3-((2-méthyl-1*H*-imidazol-1-yl)méthyl)benzonitrile oxyde (0,58g, 2,26 mmol), à 50g de SBR sur un outil à cylindres (mélangeur externe à 30°C). On incorpore ensuite 0.5 g d'anti-oxydant N-(1,3-diméthylbutyl)-N'-phényl-p-phénylènediamine. Le mélange est homogénéisé en 15 passes portefeuille.

Cette phase de mélangeage est suivie d'un traitement thermique à 120°C pendant 10 minutes sous presse à 10 bars de pression.

L'analyse par RMN ¹H a permis de déterminer un taux molaire de greffage de 0,23% et un rendement molaire de greffage de 83%.
La quantification de la fonction par RMN est réalisée en intégrant un proton Hb du motif ci-dessous : 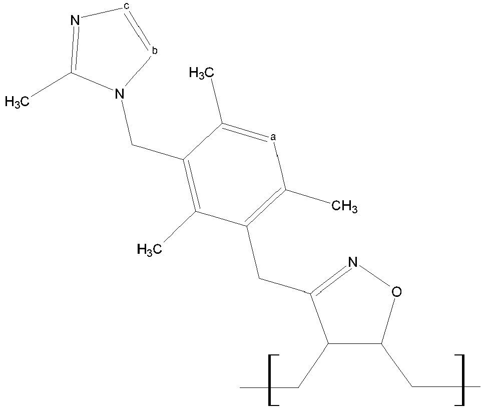
- CH (b) situé à 6.25 ppm concernant les matrices SBR

### II.4-Synthèse du composé 1,3-dipolaire 2,4,6-triméthyl-3-((2-méthyl-1H-benzo[d]imidazol-1-yl)méthyl)benzonitrile oxide :

### 11.4-1-Synthèse du 2-(chlorométhyl)-1,3,5-triméthylbenzène :

La synthèse est à l'identique de celle décrite dans le paragraphe II.2-1.

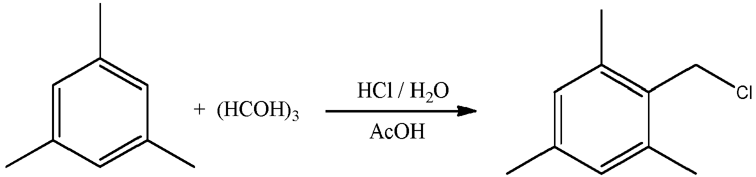

### II.4-2-Synthèse du 3-(chlorométhyl)-2,4,6-triméthylbenzaldéhyde:

La synthèse est à l'identique de celle décrite dans le paragraphe II.2-2.

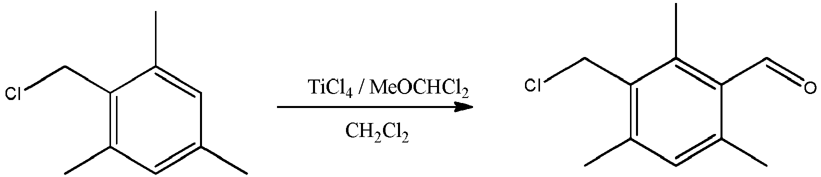

### II.4-3-Synthèse du 2,4,6-triméthyl-3-((2-méthyl-1H-benzo[d]imidazol-1-yl)méthyl)benzaldé--hyde :

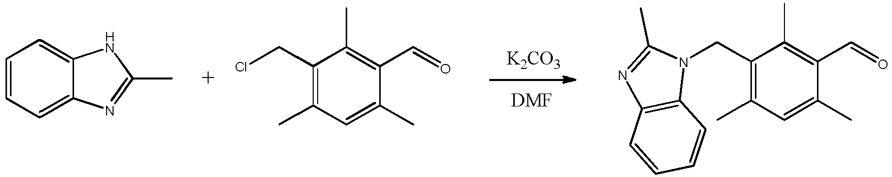

Un mélange d'aldéhyde (11,9 g, 60,5 mmol), de 2-méthylbenzimidazole (8,00 g, 60,5 mmol) et de carbonate de potassium (6,27 g, 45,4 mmol) dans le DMF (diméthylformamide, 15 ml) est agité pendant 1 heure à 80 °C et trois heures à 90 °C. Le mélange est ensuite dilué par l'eau (600ml). La phase organique est extraite par EtOAc (trois fois 150 ml) et lavée à l'eau (4 fois 75 ml). Les solvants sont évaporés sous pression réduite (36°C (T_{bain}) pour conduire à une huile brune. Celle-ci est cristallisée avec l'éther de pétrole 40/60 (15 ml) et l'éthylacétate (20 ml).
Un solide (11,70 g, 40,0 mmol, rendement 66 %) de point de fusion 118 °C est obtenu. La pureté molaire est 70 %, EtOAc - 5 % (RMN ¹H).

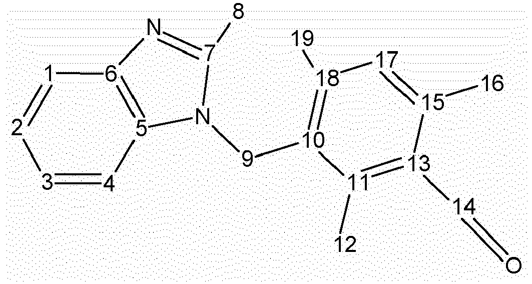

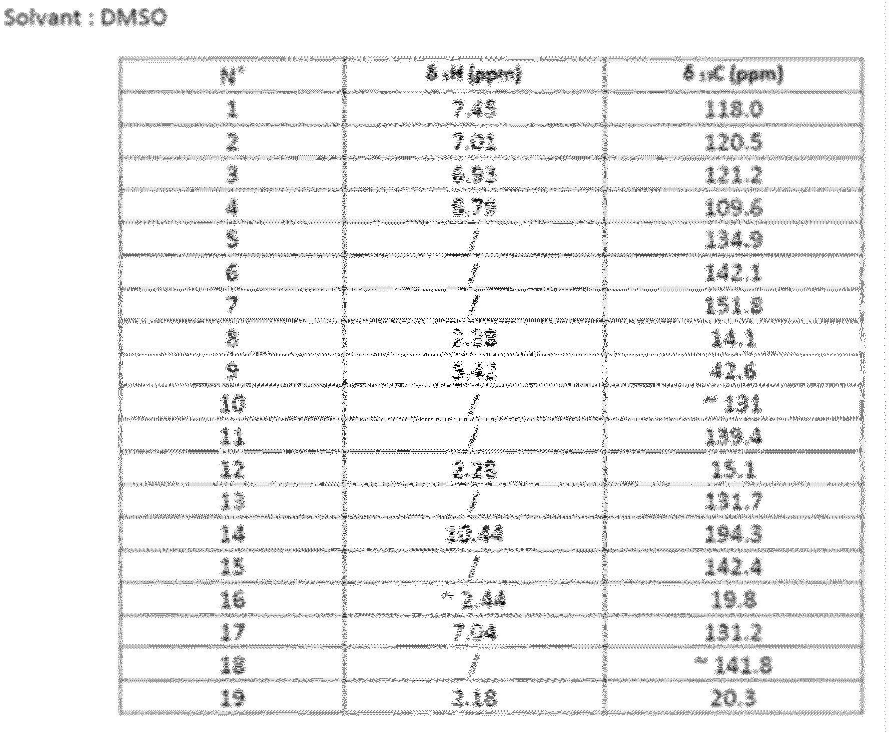

### II.4-4-Synthèse du 2,4,6-triméthyl-3-((2-méthyl-1H-benzo[d]imidazol-1-yl)méthyl)benzaldé--hyde oxime

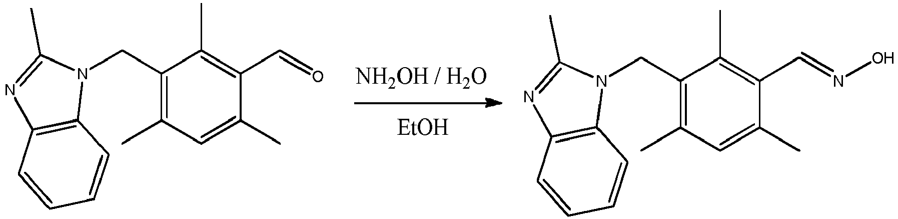

À une solution d'aldéhyde (11,5 g, 39,4 mmol) dans EtOH (80 ml) à 35 °C est ajoutée une solution d'hydroxylamine (6,14 g, 62,9 mmol, 50 % dans l'eau, Aldrich) dans EtOH (20 ml). Le milieu réactionnel est agité pendant 3,5 heures à 48-50°C. Le milieu réactionnel est ensuite refroidi jusqu'au 10-15 °C, le précipité obtenu est filtré et lavé sur le filtre par un mélange d'éthanol et d'eau (deux fois par mélange 5 ml et 10 ml) puis séché pendant 15-20 heures sous pression atmosphérique à température ambiante.
Un solide (7,95 g, 25,9 mmol, rendement 66 %) de point de fusion 248 °C est obtenu. La pureté molaire est supérieure à 80 % (RMN ¹H).

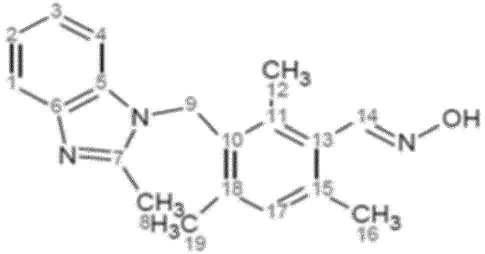

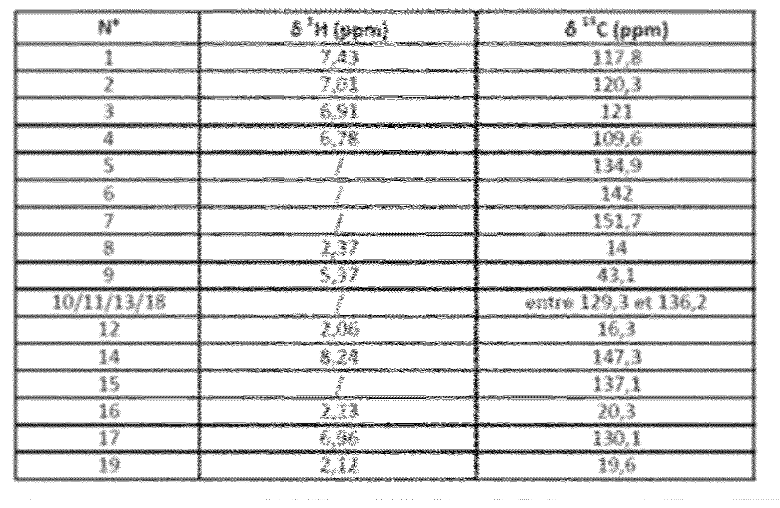

### II.4-5-Synthèse du 2,4,6-triméthyl-3-((2-méthyl-1H-benzo[d]imidazol-1-yl)méthyl)benzo--nitrile oxide :

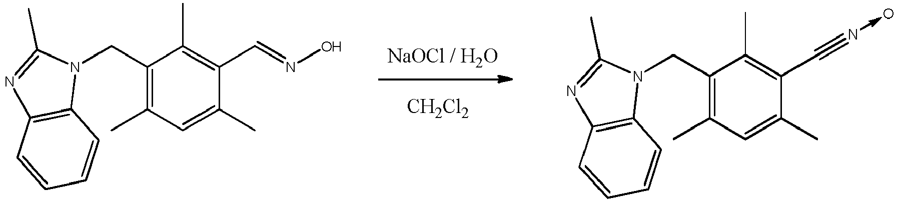

A une solution d'oxime (6,20 g, 20,2 mmol) dans le dichlorométhane (150 ml) refroidi jusqu'à 5°C est ajoutée goutte à goutte une solution aqueuse de NaOCl (6 % du chlore actif) (25,4 ml) pendant 6-8 minutes. Le milieu réactionnel est agité pendant 4,5 heures jusqu'à émulsion à 10°C. La phase organique est séparée et lavée par l'eau (3 fois par 25 ml). Après évaporation du solvant sous pression réduite (T_{bain} 22-23 ºC) jusqu'à cristallisation, de l'éther de pétrole (40/60) (10 ml) et du dichlorométhane (4 ml) sont ajoutés. La suspension est agitée pendant 10-15 minutes et le précipité est filtré, lavé sur le filtre par le mélange de CH₂Cl₂ / éther de pétrole (2 ml / 4 ml) et par l'éther de pétrole (40/60) (6 ml) et enfin séché pendant 10-15 heures sous pression atmosphérique à température ambiante.
Un solide blanc (4,85 g, 15,9 mmol, rendement 79 %) de point de fusion 142 °C est obtenu. La pureté molaire est supérieure à 71 % (RMN ¹H).
Le produit brut (4,4 g) est remis en solution dans l'acétone (100 ml), puis cette solution versée dans l'eau (500 ml), la suspension est agitée pendant 5-10 minutes. Le précipité est filtré et lavé sur le filtre par l'eau (200 ml), séché pendant 10-15 heures sous pression atmosphérique à température ambiante.
Un solide blanc (3,82 g, 12,6 mmol, rendement 62 %) de point de fusion 136,5-137,5 °C est obtenu avec une pureté de 94%mol en RMN ¹H.

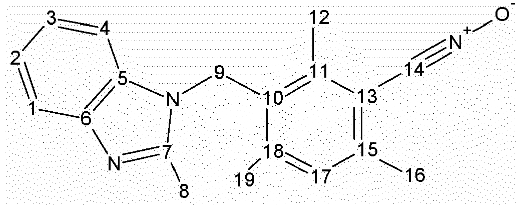

| N° | δ¹H(ppm) | δ¹³C(ppm) |
|---|---|---|
| 1 | 7.45 | 118.2 |
| 2 | 7.02 | 120.7 |
| 3 | 6.95 | 121.2 |
| 4 | 6.81 | 109.6 |
| 5 | / | 134.7 |
| 6 | / | 141.9 |
| 7 | / | 151.7 |
| 8 | 2.36 | 13.9 |
| 9 | 5.39 | 42.8 |
| 10 | / | 130.5 |
| 11 | / | entre 140.2 et 140.6 |
| 12 | 2.24 | 18.0 |
| 13 | / | 112.3 |
| 14 | / | Non Détecté |
| 15 | / | 140.9 |
| 16 | 2.34 | 19.9 |
| 17 | / | 130.2 |
| 18 | / | entre 140.2 et 140.6 |
| 19 | 21 | 19.7 |

Solvant DMSO

### II.5-Greffage du composé 1,3-dipolaire 2,4,6-triméthyl-3-((2-méthyl-1H-benzo[d]imidazol-1-yl)méthyl)benzonitrile oxide :

La figure ci-dessous représente une unité isoprène greffée :

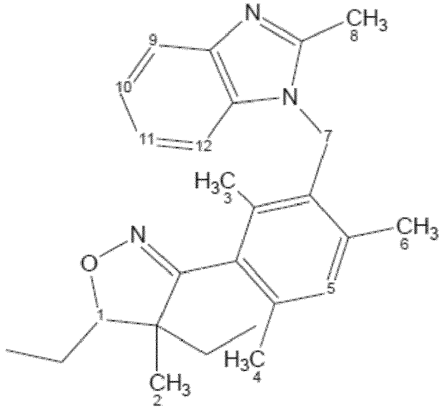

On utilise le composé 1,3-dipolaire obtenu selon le mode opératoire décrit dans le paragraphe II.4.

Le polyisoprène de synthèse (IR) avant modification contient 98% en poids de motifs 1,4-cis.
On incorpore le composé 1,3 dipolaire à raison de 1,35 g pour 100 g de IR sur un outil à cylindres (mélangeur externe à 30°C). On incorpore ensuite 1 g d'anti-oxydant N-(1,3-diméthylbutyl)-N'-phényl-p-phénylènediamine pour 100 g de IR. Le mélange est homogénéisé en 12 passes portefeuille.

Cette phase de mélangeage est suivie d'un traitement thermique à 120°C pendant 10 minutes sous presse à 10 bars de pression.
L'analyse par RMN ¹H a permis de déterminer un taux molaire de greffage de 0,20% et un rendement molaire de greffage de 71%.

### II.6-Synthèse du composé 1,3-dipolaire N-(4-((1H-imidazol-1-yl)méthyl)benzylidène)aniline oxide (Va) :

Ce composé peut être préparé selon le schéma réactionnel suivant :

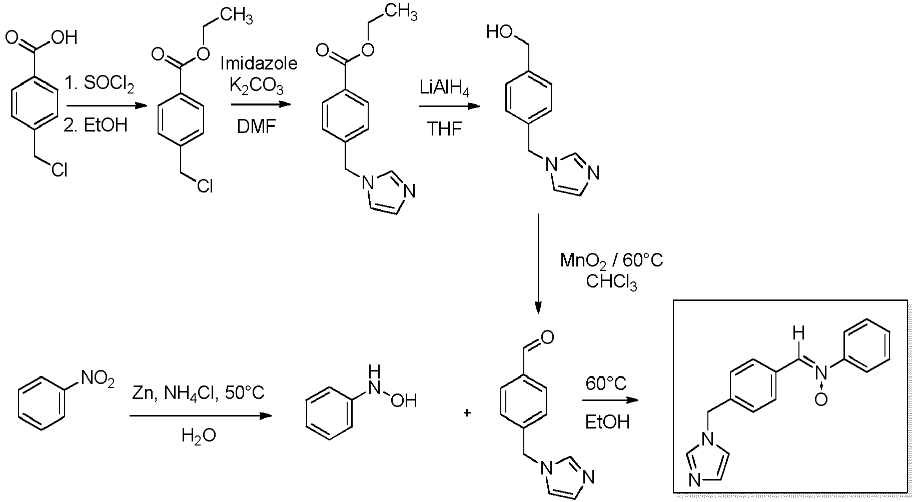

### 11.6-1-Synthèse de l'éthyl 4-(chlorométhyl)benzoate :

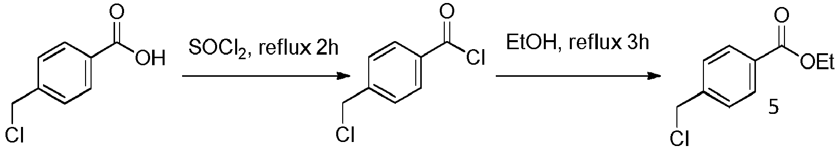

Un mélange d'acide 4-chlorométhylbenzoïque (10.64 g, 0.062 mol), de DMF (70 mg, quantité catalytique) et de SOCl₂ (25 mL, 0.346 mol) est chauffé à 75°C pendant 2 heures. La solution brune est évaporée sous pression réduite pendant 1.5 heures à 30°C. On obtient 12.9 g d'une huile brune contenant une petite quantité d'un solide. Après filtration, on ajoute en une fois de l'éthanol anhydre (60 mL). La température du milieu réactionnel s'élève immédiatement jusqu'à 40°C. La solution obtenue est ensuite portée au reflux (T_{bain} 95°C) pendant 3 heures. La réaction est contrôlée en RMN ¹H (disparition du signal Cl-CH₂-C₆H₄ à 5.8 ppm, solvant - CDCl₃). Une huile jaune (11,40 g, rendement 92 %) est obtenue après concentration sous pression réduite (40°C/15-20 mbar). La pureté molaire est supérieure à 93 % (RMN ¹H).

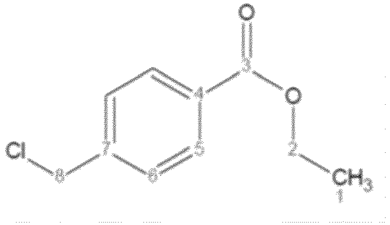

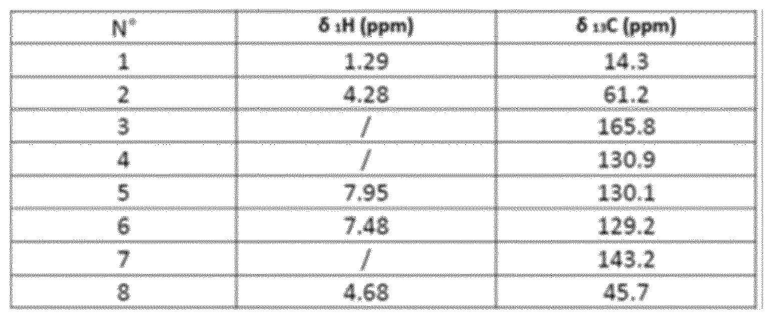

Solvant: acétone-d6

### II.6-2-Synthèse de l'éthyl 4-((1H-imidazol-1-yl)méthyl)benzoate :

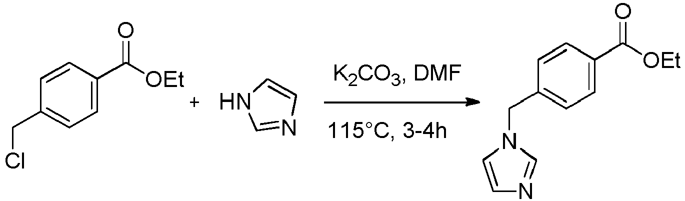

Un mélange d'ethyl 4-(chloromethyl)benzoate (13.55 g, 0.068 mol.), d'imidazole (4.62 g, 0.068 mol.) et de K₂CO₃ (12.48 g, 0.090 mol.) dans DMF (100 mL) est chauffé à 115°C (T_{bain}) pendant 3-4 heures. Après refroidissement sous atmosphère inerte (azote), le milieu réactionnel est maintenu à température ambiante pendant 10-12 heures. Le précipité jaune formé est filtré et lavé 2 fois par le DMF (30 mL). Une concentration du solvant sous pression réduite à 70-80°C permet d'obtenir environ 22 g d'huile brune qui contient DMF. Le brut réactionnel est solubilisé dans CH₂Cl₂ (150 mL) puis concentré sous pression réduite à 70-80°C. La phase organique est extraite par CH₂Cl₂ trois fois et les phases organiques rassemblées sont lavées par l'eau. L'huile brune (8.00 g, rendement 51%) est obtenue. La pureté molaire est supérieure à 81 % (RMN ¹H). Le produit a été utilisé sans purification supplémentaire.

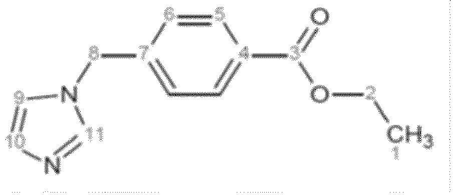

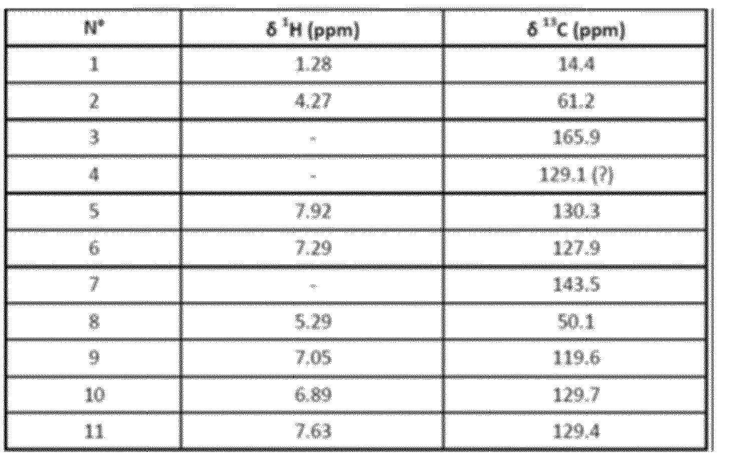

### II.6-3-Synthèse du 4-((1H-imidazol-1-yl)méthyl)phénylméthanol

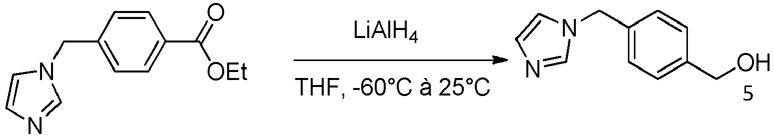

Une solution de LiAlH₄ (1.50 g, 0.039 mol) dans le THF anhydre (230 mL) est refroidi à-60°C. Une solution d'éthyl 4-((1H-imidazol-1-yl)methyl)benzoate (7.80 g, 0.028 mol, 81%mol) dans le THF anhydre (100 mL) est ajoutée sous argon pendant 15 minutes. Le milieu réactionnel est agité pendant 1 heure à -60°C, puis 10-12 heures à température ambiante. De l'eau (20 mL) est ajoutée goutte à goutte (une réaction exothermique). Le précipité formé est filtré, Le filtrat est concentré sous pression réduite. Le brut obtenu est solubilisé dans CH₂Cl₂ (100 mL) pour faire précipiter des insolubles. Après filtration et concentration sous pression réduite, une huile jaune (4,96 g, rendement 93 %) est obtenue. La pureté molaire est supérieure à 85 % (RMN ¹H).

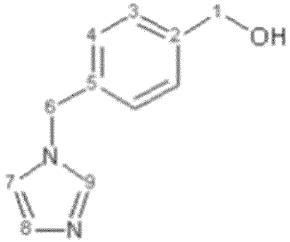

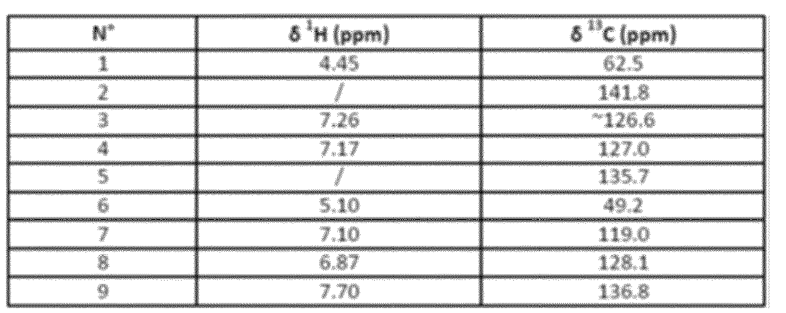

Solvant: DMSO-d6

### II.6-4-Synthèse du 4-((1H-imidazol-1-yl)méthyl)benzaldéhyde :

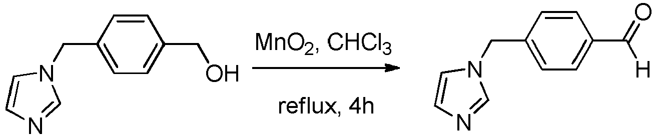

Un mélange de MnO₂ (6.88 g, 0.079 mol) et de 4-((1H-imidazol-1-yl)methyl)phenylmethanol (4.57 g, 0.021 mol, 85% mol. par RMN ¹H) dans CHCl₃ (180 mL) est agité pendant 4 heures à température au reflux. Le milieu réactionnel est refroidi jusqu'à température ambiante et est maintenu sous agitation à cette température pendant 10-12 heures. Les produits insolubles sont filtrés, le filtrat est concentré sous pression réduite. Une huile jaune (3,78 g, rendement 98 %) est obtenue après concentration sous pression réduite. La pureté molaire est supérieure à 81 % (RMN ¹H).

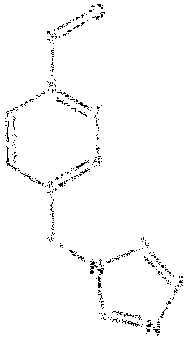

| **N°** | **δ₁H(ppm)** | **δ₁₃C(ppm)** |
|---|---|---|
| 1 | 7.37 | 136.8 |
| 2 | 6.88 | 129.1 |
| 3 | 6.74 | 118.6 |
| 4 | 5.02 | 49.4 |
| 5 | / | 142.4 |
| 6 | 7.07 | 126.7 |
| 7 | 7.62 | 129.4 |
| 8 | / | 135.2 |
| 9 | 9.74 | 190.7 |

| | | |
|---|---|---|
| Solvant: CDCl₃ | | |

### II.6-5-Synthèse de la phénylhydroxylamine :

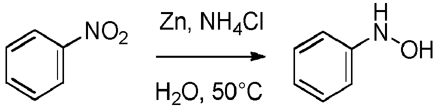

La phénylhydroxylamine a été synthétisé selon le mode opératoire décrit dans Org.Syntheses, Coll. Vol. 1, p. 445, 1941; Org.Syntheses, Coll. Vol. 3, p. 668, 1955

### II.6-6-Synthèse du N-(4-((1H-imidazol-1-yl)méthyl)benzylidène)aniline oxide :

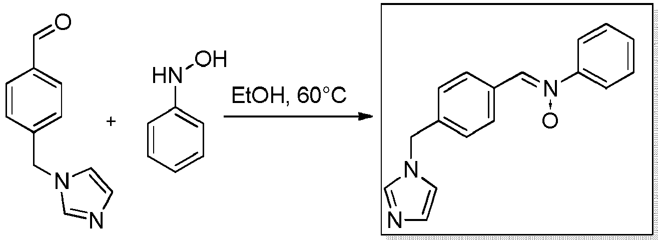

Une solution de l'aldéhyde (3.48 g, 0.015 mol, 81% mol. par ¹H RMN) et de phénylhydroxylamine (2.86 g, 0.026 mol) dans l'éthanol anhydre (20 mL) est agitée pendant 2 heures à 60°C (T_{bain}) et ensuite pendant 12 heures à température ambiante. On filtre le précipité jaune (0,249 g contenant le produit attendu). Au filtrat, on ajoute de l'eau (30 mL) sous vive agitation. Le précipité jaune alors formé est filtré après 20 minutes d'agitation et lavé par un mélange d'EtOH (10 mL) et d'eau (20 mL), puis par l'eau (50 mL). Les deux portions de solide sont réunies et séchées pendant 10-12 heures sous pression atmosphérique à température ambiante. Un solide jaune (3,71 g, rendement 89 %) de la pureté molaire supérieure à 82 % (RMN ¹H) est obtenu. Une purification supplémentaire est appliquée par agitation pendant 1.5 heures à température ambiante, filtration, lavage sur le filtre par 50 ml d'éther éthylique, séchage pendant 2 jours à température ambiante. Un solide jaune (3,04 g, rendement 78 %) de point de fusion 115-116°C est obtenu. La pureté molaire est supérieure à 88 % (RMN ¹H).

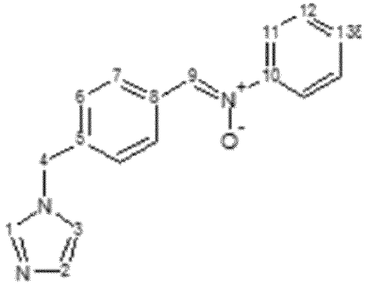

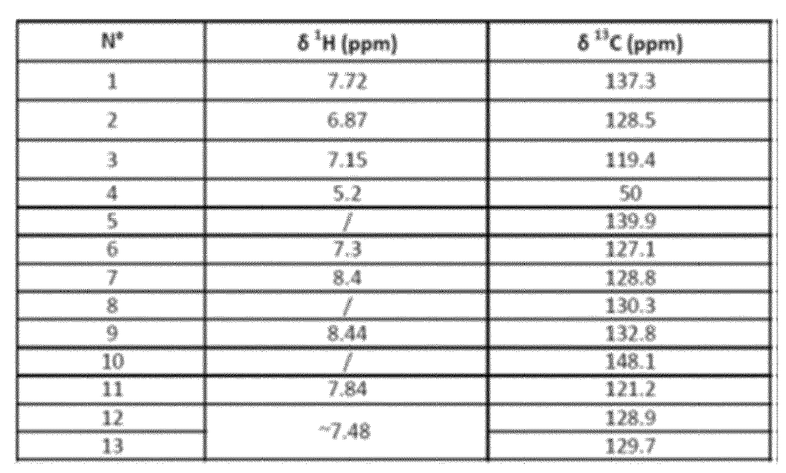

Solvant: DMSO-d6

### II.7-Préparation des compositions de caoutchouc :

Les élastomères SBR-g et IR-g sont utilisés pour la préparation des compositions de caoutchouc C-SBR-g et C-IR-g. Les élastomères SBR et IR qui ont été utilisés pour préparer les élastomères greffés SBR-g et IR-g sont utilisés sous forme non modifiée pour constituer la matrice élastomère des compositions témoins C-SBR et C-IR respectivement.

Les formulations (en pce) des compositions sont décrites dans le tableau (I). Les compositions C-SBR-g et C-IR-g sont conformes à l'invention. Les compositions C-SGR et C-IR sont des compositions témoins respectives des compositions C-SBR-g et C-IR-g.

On procède pour la fabrication de ces compositions de la manière suivante : on introduit dans un mélangeur interne (taux de remplissage final : environ 70% en volume), dont la température initiale de cuve est d'environ 110°C, successivement l'élastomère, la silice, l'agent de couplage, ainsi que les divers autres ingrédients à l'exception du système de vulcanisation. On conduit alors un travail thermomécanique (phase non-productive) en une étape, qui dure environ 5 min à 6 minutes, jusqu'à atteindre une température maximale de « tombée » de 160°C. On récupère le mélange ainsi obtenu, on le refroidit puis on incorpore du soufre et un accélérateur type sulfénamide sur un mélangeur (homo-finisseur) à 23°C, en mélangeant le tout (phase productive) pendant un temps approprié (par exemple entre 5 et 12 min).

**Tableau (I)**

| Composition | C-SBR non conforme | C-SBR-g conforme | C-IR non conforme | C-IR-g conforme |
|---|---|---|---|---|
| SBR (1) | 100 | - | - | - |
| SBR-g (2) | - | 101 | - | - |
| IR(3) | - | - | 100 | - |
| IR-g (4) | - | - | - | 101 |
| Noir de carbone N234 | 3 | 3 | 3 | 3 |
| Silice (5) | 55 | 55 | 55 | 55 |
| Silane (6) | 5.5 | 5.5 | 5.5 | 5.5 |
| Antioxydant (7) | 1.5 | 1.5 | 1 | 1 |
| Antioxydant (8) | 1 | - | 1.5 | 0.5 |
| Cire anti-ozone | 1 | 1 | 1 | 1 |
| ZnO | 2.7 | 2.7 | 2.7 | 2.7 |
| Acide stéarique | 2.5 | 2.5 | 2.5 | 2.5 |
| Sulfénamide (9) | 1.8 | 1.8 | 2 | 2 |
| Soufre | 1.5 | 1.5 | 1.3 | 1.3 |

| | | | | |
|---|---|---|---|---|
| (1) SBR : SBR avec 25% de motif styrène et 56% de motif 1,2 de la partie butadiénique (2) SBR-g : SBR modifié selon la synthèse décrite ci-dessus dans le paragraphe précédent II.3 (3) IR : polyisoprène à 98% en poids de motifs 1,4-cis (4) IR-g : IR modifié selon la synthèse décrite ci-dessus dans le paragraphe précédent II.5 (5) Silice « Zeosil 1165 MP » de la société Rhodia (type HDS) (6) TESPT (« Si69 » de la société Degussa) (7) 2,2,4-triméthyl-1,2-dihydroquinoline (8) N-(1,3-diméthylbutyl)-N'-phényl-p-phénylènediamine, de la société Flexsys (9) N-cylohexyl-2-benzothiazol-sulfénamide (« Santocure CBS » de la société Flexsys) | | | | |

Les compositions ainsi obtenues sont ensuite calandrées, soit sous forme de plaques (d'une épaisseur allant de 2 à 3 mm) ou fines feuilles de caoutchouc, pour la mesure de leurs propriétés physiques ou mécaniques, soit sous la forme de profilés directement utilisables, après découpage et/ou assemblage aux dimensions souhaitées, par exemple comme produits semi-finis pour pneumatiques, en particulier pour des bandes de roulement.
La réticulation est effectuée à 150°C. Le temps de réticulation appliqué, *t'_{c}*(90), est le temps nécessaire pour que le couple de la composition atteigne 90% du couple maximum de la composition. Les couples de la composition sont mesurés à 150°C avec un rhéomètre à chambre oscillante, selon la norme DIN 53529 - partie 3 (juin 1983). *t'_{c}*(90) est déterminé selon la norme NF T 43-015 pour chacune des compositions.

### II.8-Essais de caractérisation - Résultats :

### Essais de traction :

Ces essais de traction permettent de déterminer les contraintes d'élasticité. Sauf indication différente, ils sont effectués conformément à la norme française NF T 46-002 de septembre 1988. Un traitement des enregistrements de traction permet également de tracer la courbe de module en fonction de l'allongement. On mesure en première élongation le module sécant nominal calculé en se ramenant à la section initiale de l'éprouvette (ou contrainte apparente, en MPa) à 100% d'allongement noté MSA100. Toutes ces mesures de traction sont effectuées dans les conditions normales de température (23 ± 2°C) selon la norme NF T 46-002.

### Propriétés dynamiques :

Les propriétés dynamiques sont mesurées sur un viscoanalyseur (Metravib VA4000), selon la norme ASTM D 5992-96. On enregistre la réponse d'un échantillon de composition vulcanisée (éprouvette cylindrique de 4 mm d'épaisseur et de 400 mm² de section), soumis à une sollicitation sinusoïdale en cisaillement simple alterné, à la fréquence de 10Hz, dans les conditions normales de température (23°C) selon la norme ASTM D 1349-99, ou selon les cas à une température différente (100°C). On effectue un balayage en amplitude de déformation de 0,1% à 100% (cycle aller), puis de 100% à 0,1% (cycle retour). Les résultats exploités sont le module complexe de cisaillement dynamique (G*) à 25% de déformation, le facteur de perte tan(δ) et l'écart de module (ΔG*) entre les valeurs à 0,1 et 100% de déformation (effet Payne). Pour le cycle retour, on indique la valeur maximale de tan(δ) observée, noté tan(δ)max.

Les résultats sont consignés dans le tableau (II) ci-après.

**Tableau (II)**

| **Composition** | C-SBR | C-SBR-g | C-IR | C-IR-g |
|---|---|---|---|---|
| **Propriétés à cuit** | | | | |
| MSA100 à 23°C | 2.83 | 3.19 | 1.84 | 3.72 |
| tanδ max à 23°C | 0.28 | 0.17 | 0.23 | 0.08 |
| ΔG* à 23°C | 4 | 0.93 | 3.27 | 0.59 |
| G* à 100°C | 1.61 | 1.63 | 1.35 | 1.45 |
| tanδ max à 100°C | 0.13 | 0.08 | 0.13 | 0.06 |

Les compositions C-SBR-g et C-IR-g présentent à 23°C un module MSA100 à 23°C qui est bien supérieur à celui des compositions témoin respectives C-SBR et C-IR. Cette hausse de rigidité à cuit est obtenue bien qu'une diminution très significative de l'hystérèse à 23°C soit également observée pour C-SBR-g et C-IR-g en comparaison à leur témoin respectif C-SBR et C-IR. L'augmentation de la rigidité à cuit est d'autant plus remarquable que la baisse d'hystérèse est très forte.
Un bon comportement routier d'un pneumatique étant généralement associé à une forte rigidité à cuit de la composition qui constitue sa bande de roulement, ce résultat augure un bon comportement routier d'un pneumatique ayant une bande de roulement comportant une composition C-SBR-g ou C-IR-g.

Par ailleurs il est observé que les compositions selon l'invention C-SBR-g et C-IR-g conservent un niveau de rigidité à cuit à 100°C comparable à celui des compositions témoin respectives C-SBR et C-IR. Ces résultats présagent d'une polyvalence en température de la composition de caoutchouc conforme à l'invention. En effet on peut attendre qu'une bande de roulement contenant la composition C-SBR-g ou C-IR-g permette au pneumatique d'avoir un comportement routier au moins tout aussi bon que ne le ferait la composition témoin C-SBR ou C-IR, lors de conditions de roulage plus extrêmes, notamment pour des pneumatiques de voiture sport roulant à haute vitesse.

## Revendications

1. Procédé pour modifier par une réaction de greffage un polymère insaturé qui présente au moins une insaturation, lequel procédé comprend la réaction d'un composé 1,3-dipolaire sur au moins une insaturation du polymère insaturé, le composé 1,3-dipolaire étant choisi dans le groupe constitué par les oxydes de nitrile, les imines de nitrile et les nitrone et répondant à la formule (I)
Q-A-B (I)
dans laquelle :
Q. contient un motif -C≡N→O, -C≡N→N- ou -C=N(→O)-,
A est un atome ou un groupe d'atomes reliant Q à B,
B comprend un cycle répondant à la formule (II) 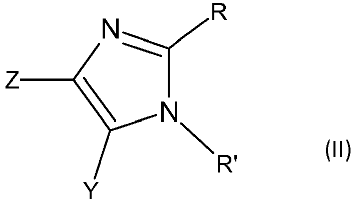
dans laquelle :
3 des 4 symboles Z, Y, R et R' identiques ou différents représentent chacun un atome ou un groupe d'atomes,
et le quatrième symbole Z, Y, R ou R' désigne un rattachement direct à A,
dans lequel :
A contenant jusqu'à 20 atomes de carbone est un groupe aliphatique ou un groupe aromatique,
R' désigne le rattachement direct à A,
R représente un atome d'hydrogène ou un groupe alkyle,
Z et Y sont chacun un atome d'hydrogène.

2. Procédé selon la revendication 1 dans lequel Q contient un motif -C≡N→O.

3. Procédé selon la revendication 2 dans lequel Q comporte le motif répondant à la formule (III) : 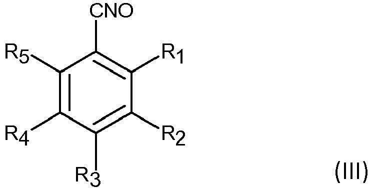 dans laquelle :
quatre des cinq symboles R1 à R5 identiques ou différents, sont chacun un atome ou un groupe d'atomes, et le cinquième symbole désigne un rattachement direct à A, sachant que R1 et R5 sont tous les deux différents de H.

4. Procédé selon la revendication 3 dans lequel R1, R3 et R5 sont chacun un groupe alkyle de 1 à 6 atomes de carbone.

5. Procédé selon la revendication 4 dans lequel R1, R3 et R5 sont chacun un méthyle ou éthyle.

6. Procédé selon la revendication 5, dans lequel le composé 1,3-dipolaire est le 2,4,6-triméthyl-3-((2-méthyl-1*H*-imidazol-1-yl)méthyl)benzo-nitrile oxyde ou le 2,4,6-triéthyl-3-((2-méthyl-1*H*-imidazol-1-yl)méthyl)benzo-nitrile oxyde.

7. Procédé selon la revendication 1 dans lequel Q. contient un motif -C=N(→O)-.

8. Procédé selon la revendication 7 dans lequel Q comporte le motif répondant à la formule (IV) ou (V) 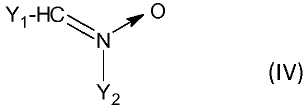 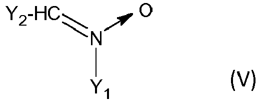 dans laquelle :
Y₁ est un groupe aliphatique ou un groupe aromatique contenant de 6 à 20 atomes de carbone,
Y₂, comportant un rattachement direct à A, est un groupe aliphatique ou un groupe aromatique comportant sur son noyau benzénique le rattachement direct à A.

9. Procédé selon la revendication 8 dans lequel le composé 1,3-dipolaire est de formule (IVa), (IVb), (Va) ou (Vb).

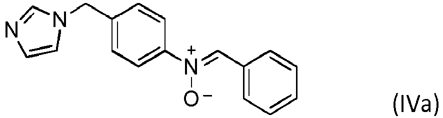

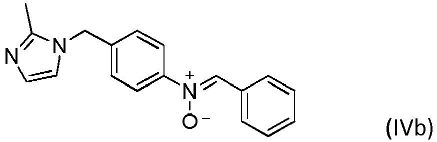

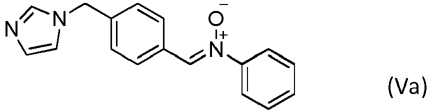

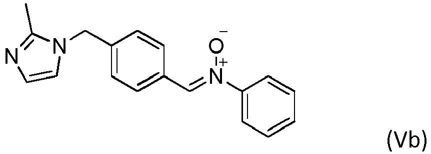

10. Procédé selon l'une quelconque des revendications 1 à 9 dans lequel on fait réagir le composé 1,3-dipolaire selon une stœchiométrie comprise entre 0 et 3 équivalents molaires de cycle imidazole pour 100 moles d'unités monomères constituant le polymère.

11. Procédé selon l'une quelconque des revendications 1 à 10 dans lequel le polymère insaturé est un polymère diénique.

12. Polymère susceptible d'être obtenu par le procédé défini selon l'une quelconque des revendications 1 à 11.

13. Composé 1,3-dipolaire défini selon l'une quelconque des revendications 2 à 6.

14. Composé 1,3-dipolaire défini selon la revendication 8 dans lequel Y₁ est un groupe alkyle ou un groupe aromatique contenant de 6 à 20 atomes de carbone et dans lequel Y₂, est un groupe aromatique contenant un noyau benzénique et comportant sur son noyau benzénique un rattachement direct à A et dans lequel A est un groupe alkylène contenant de 1 à 20 atomes de carbone ou un groupe arylène.

15. Composé 1,3-dipolaire défini selon la revendication 9.

## Patentansprüche

1. Verfahren zur Modifizierung eines ungesättigten Polymers, das mindestens eine Ungesättigtheit aufweist, durch eine Pfropfreaktion, bei dem man eine 1,3-dipolare Verbindung mit mindestens einer Ungesättigtheit des ungesättigten Polymers zur Reaktion bringt, wobei die 1,3-dipolare Verbindung aus der Gruppe bestehend aus Nitriloxiden, Nitriliminen und Nitronen ausgewählt ist und der Formel (I) entspricht:
Q-A-B (I),
wobei:
Q eine Einheit -C≡N→O, -C≡N→N- oder -C=N(→O)-umfasst,
A für ein Atom oder eine Gruppe von Atomen, das bzw. die Q mit B verbindet, steht,
B einen Ring der Formel (II) umfasst: 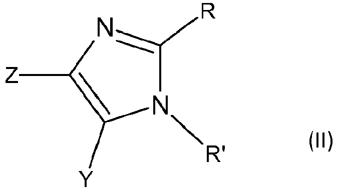
wobei:
3 der 4 Symbole Z, Y, R und R' gleich oder verschieden sind und jeweils für ein Atom oder eine Gruppe von Atomen stehen
und das vierte Symbol Z, Y, R bzw. R' eine direkte Bindung an A bezeichnet,
wobei:
A bis zu 20 Kohlenstoffatome enthält und eine aliphatische Gruppe oder eine aromatische Gruppe ist,
R' die direkte Bindung an A bezeichnet,
R für ein Wasserstoffatom oder eine Alkylgruppe steht,
Z und Y jeweils für ein Wasserstoffatom stehen.

2. Verfahren nach Anspruch 1, wobei Q eine Einheit -C≡N→O enthält.

3. Verfahren nach Anspruch 2, wobei Q die Einheit der Formel (III) umfasst: 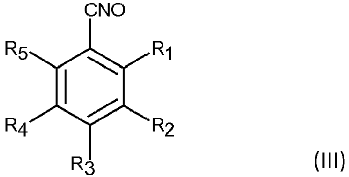 wobei:
vier der fünf Symbole R1 bis R5 gleich oder verschieden sind und jeweils für ein Atom oder eine Gruppe von Atomen stehen und das fünfte Symbol eine direkte Bindung an A bezeichnet, mit der Maßgabe, dass R1 und R5 beide von H verschieden sind.

4. Verfahren nach Anspruch 3, wobei R1, R3 und R5 jeweils für eine Alkylgruppe mit 1 bis 6 Kohlenstoffatomen stehen.

5. Verfahren nach Anspruch 4, wobei R1, R3 und R5 jeweils für ein Methyl oder Ethyl stehen.

6. Verfahren nach Anspruch 5, wobei es sich bei der 1,3-dipolaren Verbindung um 2,4,6-Trimethyl-3-((2-methyl-1*H*-imidazol-1-yl)methyl)benzonitriloxid oder 2,4,6-Triethyl-3-((2-methyl-1*H*-imidazol-1-yl)methyl)benzonitriloxid handelt.

7. Verfahren nach Anspruch 1, wobei Q eine Einheit -C=N(→O)- umfasst.

8. Verfahren nach Anspruch 7, wobei Q die Einheit der Formel (IV) oder (V) umfasst: 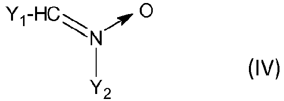 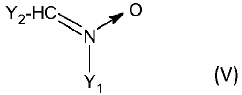 wobei:
Y₁ für eine aliphatische Gruppe oder eine aromatische Gruppe mit 6 bis 20 Kohlenstoffatomen steht,
Y₂ eine direkte Bindung an A umfasst und für eine aliphatische Gruppe oder eine aromatische Gruppe mit der direkten Bindung an A an ihrem Benzolkern steht.

9. Verfahren nach Anspruch 8, wobei die 1,3-dipolare Verbindung die Formel (IVa), (IVb), (Va) oder (Vb) aufweist:

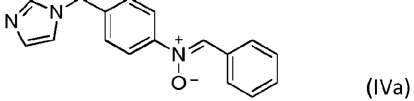

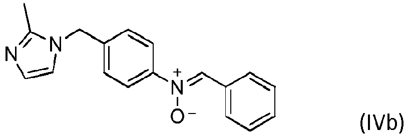

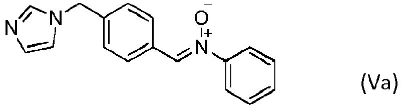

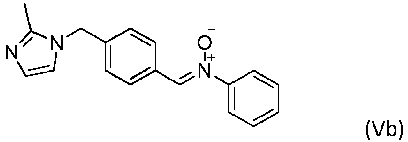

10. Verfahren nach einem der Ansprüche 1 bis 9, wobei man die 1,3-dipolare Verbindung gemäß einer Stöchiometrie zwischen 0 und 3 Moläquivalenten Imidazolring pro 100 mol Monomereinheiten, aus denen das Polymer aufgebaut ist, zur Reaktion bringt.

11. Verfahren nach einem der Ansprüche 1 bis 10, wobei es sich bei dem ungesättigten Polymer um ein Dienpolymer handelt.

12. Polymer, das durch das Verfahren gemäß einem der Ansprüche 1 bis 11 erhältlich ist.

13. 1,3-Dipolare Verbindung gemäß einem der Ansprüche 2 bis 6.

14. 1,3-Dipolare Verbindung gemäß Anspruch 8, wobei Y₁ für eine Alkylgruppe oder eine aromatische Gruppe mit 6 bis 20 Kohlenstoffatomen steht und wobei Y₂ für eine aromatische Gruppe mit einem Benzolkern und mit einer direkten Bindung an A an ihrem Benzolkern steht und wobei A für eine Alkylengruppe mit 1 bis 20 Kohlenstoffatomen oder eine Arylengruppe steht.

15. 1,3-Dipolare Verbindung gemäß Anspruch 9.

## Claims

1. Process for modifying, by a grafting reaction, an unsaturated polymer which exhibits at least one unsaturation, which process comprises the reaction of a 1,3-dipolar compound with at least one unsaturation of the unsaturated polymer, the 1,3-dipolar compound being selected from the group consisting of nitrile oxides, nitrile imines and nitrones and corresponding to the formula (I):
Q-A-B (I)
in which:
Q contains a -C≡N→O, -C≡N→N- or -C=N(→O)- unit,
A is an atom or a group of atoms connecting Q to B,
B comprises a ring corresponding to the formula (II): 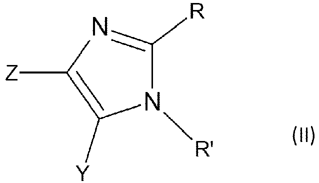
in which:
three of the four symbols Z, Y, R and R', which are identical or different, each represent an atom or a group of atoms,
and the fourth symbol Z, Y, R or R' denotes a direct attachment to A
in which
A containing up to 20 carbon atoms is an aliphatic group or an aromatic group,
R' denotes a direct attachment to A,
R represents a hydrogen atom or an alkyl group,
Z and Y are each a hydrogen atom.

2. Process according to Claim 1, in which Q contains a -C≡N→O unit.

3. Process according to Claim 2, in which Q comprises the unit corresponding to the formula (III): 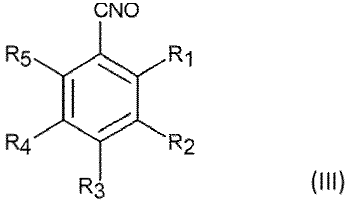 in which:
four of the five symbols R₁ to R₅, which are identical or different, are each an atom or a group of atoms, and the fifth symbol denotes a direct attachment to A, it being known that R₁ and R₅ are both other than H.

4. Process according to Claim 3, in which R₁, R₃ and R₅ are each an alkyl group of 1 to 6 carbon atoms.

5. Process according to Claim 4, in which R₁, R₃ and R₅ are each a methyl or ethyl.

6. Process according to Claim 5, which compound is 2,4,6-trimethyl-3-((2-methyl-1*H-*imidazol-1-yl)methyl)benzonitrile oxide or 2,4,6-triethyl-3-((2-methyl-1*H*-imidazol-1-yl)methyl)benzonitrile oxide.

7. Process according to Claim 1, in which Q contains a -C=N(→O)- unit.

8. Process according to Claim 7, in which Q comprises the unit corresponding to the formula (IV) or (V): 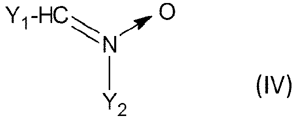 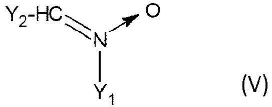 in which:
Y₁ is an aliphatic group or an aromatic group containing from 6 to 20 carbon atoms,
Y₂, comprising a direct attachment to A, is an aliphatic group or an aromatic group comprising on its benzene nucleus the direct attachment to A.

9. Process according to Claim 8 in which the 1,3-dipolar compound is of formula (IVa), (IVb), (Va) or (Vb) :

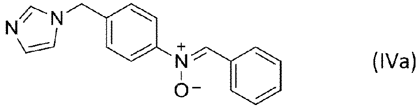

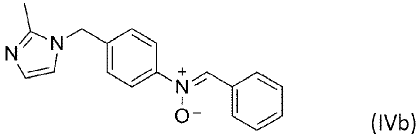

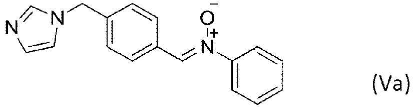

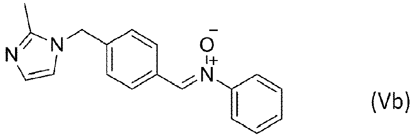

10. Process according to any one of Claims 1 to 9, in which the 1,3-dipolar compound is reacted according to a stoichiometry of between 0 and 3 molar equivalents of imidazole ring per 100 moles of monomer units constituting the polymer.

11. Process according to any one of Claims 1 to 10, in which the unsaturated polymer is a diene polymer.

12. Polymer capable of being obtained by the process defined according to any one of Claims 1 to 11.

13. 1,3-dipolar compound defined according to any one of Claims 2 to 6.

14. 1,3-dipolar compound defined according to Claim 8 in which Y₁ is an alkyl group or an aromatic group containing from 6 to 20 carbon atoms and in which Y₂, is an aromatic group containing a benzene nucleus and comprising on its benzene nucleus a direct attachment to A and in which A is an alkylene group containing from 1 to 20 carbon atoms or an arylene group.

15. 1,3-dipolar compound defined according to Claim 9.
